Europäisches Patentamt

European Patent Office

Office européen des brevets

(19)

(11) Veröffentlichungsnummer: **0 321 754 B1**

## (12) EUROPÄISCHE PATENTSCHRIFT

(45) Veröffentlichungstag der Patentschrift: **04.03.92**

(51) Int. Cl.⁵: **A61M 1/34**

(21) Anmeldenummer: **88120054.7**

(22) Anmeldetag: **01.12.88**

54 **Blutbehandlungsvorrichtung.**

(30) Priorität: **19.12.87 DE 3743272**

(43) Veröffentlichungstag der Anmeldung:
**28.06.89 Patentblatt 89/26**

(45) Bekanntmachung des Hinweises auf die
Patenterteilung:
**04.03.92 Patentblatt 92/10**

(84) Benannte Vertragsstaaten:
**ES FR IT**

(56) Entgegenhaltungen:
**DE-A- 2 703 188**
**DE-A- 3 132 790**
**FR-A- 2 434 624**

**BIOMEDIZINISCHE TECHNIK, Band 21, Nr. 10,
1976, Seiten 302-306; H. HOLTZ et al.:
"Technische Aspekte der klinischen Diafiltration"**

(73) Patentinhaber: **Fresenius AG
Gluckensteinweg 5
W-6380 Bad Homburg v.d.H.(DE)**

(72) Erfinder: **Kalepky, Ulrich, Dr.
Peter-Thumb-Strasse 11
W-7808 Waldkirch(DE)**
Erfinder: **Walter, Raimond, Dipl.-Phys.
Richard-Strauss-Strasse 1
W-8721 Röthlein(DE)**

(74) Vertreter: **Dr. Fuchs, Dr. Luderschmidt
Dipl.-Phys. Seids, Dr. Mehler Patentanwälte
Abraham-Lincoln-Strasse 7
W-6200 Wiesbaden(DE)**

## Beschreibung

Die Erfindung betrifft eine Blutbehandlungsvorrichtung nach dem Oberbegriff des Anspruchs 1. Eine derartige Blutbehandlungsvorrichtung ist aus der DE-A-3 132 790 bekannt.

Bei der Hämodiafiltration wird eine Dialyse nierenkranker Patienten dadurch durchgeführt, daß eine Substitutionsflüssigkeit dem Blut des Patienten zugeführt wird, die durch die Membran eines Hämodiafilters in Form eines Ultrafiltrats wieder entnommen wird, wobei zugleich die zu entfernenden Stoffwechselprodukte abgetrennt werden.

Die Ultrafiltration und die Substitution müssen gegeneinander ausgewogen sein, d. h. die zu- und abgeförderten Flüssigkeitsmengen müssen gegeneinander bilanziert werden.

Aus den DE-OSen 25 52 304 und 26 54 396 sind Hämodiafiltrationsvorrichtungen bekannt, die jeweils einen Behälter für die Substituatlösung und einen Behälter für das Ultrafiltrat aufweisen. Diese Behälter sind jeweils auf einer Wägeanordnung angeordnet, deren Signale jeweils in einer entsprechenden Komparatorschaltung miteinander verglichen werden und anschließend eine Steuervorrichtung antreiben, die die Bilanzierung der gesamten Flüssigkeiten vornimmt.

Im Gegensatz hierzu schlägt die DE-OS 26 29 717 nur eine Waage bei einer solchen Hämodiafiltrationsanordnung vor.

Schließlich ist aus der DE-OS 31 32 790 eine Hämodiafiltrationsvorrichtung bekannt, die ebenfalls eine Waage aufweist, wobei das Wägeergebnis einer Steuervorrichtung zugeführt wird, die eine einzige Schlauchpumpe antreibt, in die sowohl die Ultrafiltratschlauchleitung als auch die Substituatschlauchleitung eingelegt ist.

Eine geeignete Vorrichtung zur Bereitstellung der Dialysierflüssigkeit ist in der DE-OS 28 38 414 angegeben.

Sämtliche Vorrichtungen beschreiben nicht, wie sie einen ersten auftretenden Fehler sicher erkennen können. Dies ist insbesondere bei Schlauchpumpen notwendig, die in ihren Förderraten typischerweise um ± 10% schwanken. Bei einer Substituatmenge von 10 kg entspricht dies schon einem Bilanzierungsfehler von 1 kg, der bei einem Ausfall der Waage unbemerkt auftreten kann.

Noch tragischer ist ein erster Fehler der Waage (elektrisch oder mechanisch), da der Waagenwert zur Steuerung der Substituatpumpe benutzt wird.

Erfindungsgemäß soll daher die Hämodiafiltrationsanordnung den Anforderungen der DIN VDE0750 Teil 1 und Teil 206 entsprechen.

Insofern liegt der Erfindung die Aufgabe zugrunde, ein Verfahren und eine Vorrichtung zum Erkennen eines ersten Fehlers bei einer Blutbehandlung zur Verfügung zu stellen, bei denen Bilanzierungsfehler von typisch 1% sicher erkannt werden können, auch wenn ein an der Steuerung beteiligtes Gerät ausfällt.

Diese Aufgabe wird durch die kennzeichnenden Merkmale des Anspruchs 1 gelöst.

Damit können sicher Fehler in der Flüssigkeitsbilanzierung bei der Blutbehandlung, insbesondere bei der Hämodiafiltration erkannt werden, die größer als 1% vom Soll sind.
Ausgestaltungen der Erfindung sind in den Unteransprüchen angegeben.

Erfindungsgemäß wird zunächst vorteilhafterweise als Ultrafiltrationseinheit eine volumetrisch arbeitende Ultrafiltratpumpe eingesetzt, die durch eine hohe Ganggenauigkeit ausgezeichnet ist, beispielsweise 1% oder geringer. Zu solchen Pumpen gehören u.a. taktgesteuerte Membranpumpen, die je Hub eine bestimmte Menge Flüssigkeit fördern, beispielsweise 1 - 2 ml/Hub. Eine solche Pumpe zeichnet sich weiterhin durch eine geringe Fehleranfälligkeit bzw. Ausfallanfälligkeit aus.

Des weiteren wird erfindungsgemäß als Wägeeinrichtung vorteilhafterweise eine Waage eingesetzt, deren Ganggenauigkeit wenigstens 1 %o beträgt. Derartige Waagen weisen weiterhin während der Behandlungsdauer von drei bis vier Stunden keine nennenswerte Drift auf, müssen also regelmäßig nicht nachgeeicht werden.

Im übrigen wird vorteilhafterweise eine solche Waage erfindungsgemäß eingesetzt, die ihre Signale bereits elektronisch aufgearbeitet zur Verfügung stellt, so daß die Signale unmittelbar in eine elektronische Auswerteeinheit eingegeben werden können.

Nach dem erfindungsgemäßen Verfahren wird vor dem Beginn der Dialysebehandlung zunächst eine Initialprüfung der Waage und der Ultrafiltrationspumpe durchgeführt. Hierbei wird die erfindungsgemäß bevorzugt eingesetzte Membranpumpe, deren Pumpvolumen/Hub bekannt ist, mit einer bestimmten Anzahl von Pumptakten, beispielsweise 40, angesteuert, woraufhin bei einem Pumpvolumen von 1 ml entsprechend 40 ml an die Waage abgegeben werden. Dieser Sollwert von 40 ml wird mit dem Wägeergebnis verglichen, woraufhin erst eine Freigabe dieser beiden Komponenten erfolgt, wenn die beiden Ergebnisse innerhalb des vorgegebenen Fehlerwertes übereinstimmen.

Desgleichen wird auch die Substituatpumpe einer Initialprüfung sowie einer Eichung unterzogen. Dies kann in entsprechender Weise dadurch erfolgen, daß das Wägeergebnis mit der Pumprate verglichen wird. Die Pumprate kann bei einer Membranpumpe durch die Hubzahl und bei einer peristaltischen Pumpe durch die Umdrehungszahl der Pumpachse vorgegeben sein.

Als Substituatpumpe kann auch eine peristaltische Schlauchpumpe eingesetzt werden, die infolge ihrer hohen Gangungenauigkeit von ± 10% zu Beginn des Betriebs dadurch geeicht wird, daß die Zahl der Umdrehungen der Schlauchpumpe mit der von der Waage geförderten Flüssigkeitsmenge korreliert wird. Die ermittelte Förderrate je Umdrehung der Pumpe wird in die Auswerte-und Steuereinheit, nicht jedoch in die Überwachungseinheit eingegeben.

Unabhängig hiervon wird eine Überwachung der Umdrehung der Schlauchpumpe durchgeführt, dies kann beispielsweise dadurch erfolgen, daß auf der Drehachse der Pumpe eine Kodierungsscheibe vorgesehen ist, die eine Vielzahl von Schlitzen aufweist, die mit einer optoelektronischen Überwachungseinheit in Verbindung stehen, beispielsweise einer lichtemittierenden Diode und einem Lichtsensor, der die durchgelassenen Lichtpulse je Zeiteinheit ermittelt, d.h. es wird eine bestimmte Anzahl von Lichtsignalen mit einer bestimmten Fördermenge in Beziehung gesetzt.

Danach werden drei weitere Hauptparameter, nämlich die zu ultrafiltrierende Flüssigkeitsmenge $Q_{UF}$ und die Behandlungsdauer T, in die Auswerte- und Steuereinheit als Sollwerte eingegeben. Die Menge der auszutauschenden Substitutionsflüssigkeit $Q_{Sub}$ kann ebenfalls eingegeben werden, wird jedoch vorteilhafterweise von der Betriebseinheit, wie nachstehend erläutert, ermittelt und dem Schutzsystem mitgeteilt.

Diese drei Hauptparameter für die Bilanzierung werden auf folgendem Weg gebildet :
Die Menge der auszutauschenden Substitutionsflüssigkeit beispielsweise vier Beutel a 5 Liter Substitutionslösung, wird von der Betriebseinheit über die Waage ermittelt. Nachdem das Substituat eingerichtet ist und quittiert wurde, übernimmt das Betriebssystem das Bruttogewicht auf der Waage, wobei das Gewicht der Leerbehälter wegtariert worden ist. Es ermittelt dabei unter Berücksichtigung der Beuteltara, des Gewichts des Substituatsschlauches und der Sicherheitsrestmenge je Beutel die tatsächliche Substitutionsflüssigkeitsmenge, wobei diese nach Abzug der im Initialtest verworfenen Menge in der Anzeige dargestellt wird. Dieses Datum $Q_{Sub}$ wird vom Bediener auf Richtigkeit überprüft und anschließend quittiert.

Hierauf erfolgt die Eingabe der Behandlungsdauer T in Stunden und Minuten.

Danach wird die Menge $Q_{UF}$ der durch die Ultrafiltration dem Patienten zu entziehenden Flüssigkeit eingegeben. Diese Daten werden vom Bediener bei der Eingabe im Display auf Richtigkeit überprüft und anschließend quittiert.

Mit der Start-Taste wird der Behandlungsbeginn ausgelöst, wobei die vorher eingegebenen drei Parameter dem Schutzsystem übergeben werden.

Vorteilhafterweise weist die erfindungsgemäße Hämofiltrationsvorrichtung zwei Systeme in Form von Mikroprozessoren auf, wobei das eine System als reines Betriebssystem ausgebildet ist und das andere System ein Schutzsystem darstellt. Beide Systeme errechnen unabhängig voneinander die sich aus den drei Parametern ergebenen restlichen für die Steuerung und Überwachung benötigten Parameter, wie die Substituatrate, Filtratrate und Entzugsrate, um nach einem Kreuzvergleich die Behandlung freizugeben.

Die Zahlenwerte (Binärwerte) für die Raten werden für beide Systeme unterschiedlich bewertet. Das Betriebssystem bildet aus der Filtratrate (ml/min.) eine Frequenz, mit der die Ultrafiltratpumpe entsprechend angesteuert und getaktet betrieben wird.

Vorteilhafterweise fördert die Ultrafiltratpumpe je Ansteuerimpuls 2 ml ± 1%. Die Ansteuerfrequenz wird mit Hilfe eines Timers gebildet, dessen Preset-Werte in einer Softwaretabelle in 1 ml-Stufen abgelegt sind. Mit einem entsprechenden Softwareprogramm wird also der Binärwert der Ultrafiltratrate in einen Zählerwert gewandelt und in den Baustein geschrieben. Der Timer erzeugt eine der Ultrafiltratrate proportionale Frequenz, die am Ausgang zur Verfügung steht.

Um ein gleiches Testverhältnis zu gewährleisten, ist dem Timerausgang eine Monostufe nachgeschaltet. Da das Schutzsystem ebenfalls die Ultrafiltratrate kennt, kann die Rückmeldung der Ultrafiltratpumpe in vorgegebenen Zeitabständen auf umgekehrten Weg überprüft werden. Die Rückmeldeimpulse werden auf einen Zähler gegeben, der je Zeiteinheit die Impulsmenge proportional zur Filtratrate beinhaltet.

Erfindungsgemäß wird also vom Schutzsystem die Ultrafiltratleistung der Ultrafiltratpumpe aus der je Zeiteinheit abgegebenen Taktzahl der Ultrafiltratpumpe errechnet. Desgleichen wird das Wägeergebnis von der Waage entgegengenommen. Dieses aktuelle Wägeergebnis entspricht der tatsächlich zu diesem Zeitpunkt aufgenommenen Ultrafiltrationsmenge. Diese Ultrafiltrationsmenge wird verglichen mit dem zu Beginn eingegebenen Sollwert, d.h. mit der Sollultrafiltrationsrate.

Vorteilhafterweise kann dieser Ultrafiltrationswert noch mit dem Rückmeldeergebnis der Substituatpumpe verglichen werden. So wird aus der von der Substituatpumpe rückgemeldeten Umdrehungszahl die abgeförderte Substituatmenge $Q_{Sub}$ gebildet, die von der je Zeiteinheit ultrafiltrierten Menge $Q_{UF}$ subtrahiert wird, worauf sich der Ultrafiltrationswert/Zeiteinheit ergibt. Dieser errechnete Ultrafiltrationswert muß mit dem von der Waage an das Schutzsystem abgegebenen Ultrafiltrationswert übereinstimmen. Sollte dies innerhalb der vorgegebenen Fehlergrenzen, beispielsweise 1% nicht der Fall sein, so wird ein entsprechender Alarm ausgelöst und die gesamte Vorrichtung stillgesetzt.

Einen Fehler bei der Ultrafiltration kann das Schutzsystem aufgrund der Kenntnis des UF-Sollwertes, mit dem das Betriebssystem die UF-Pumpe ansteuert, über die akkumulierten UF-Pumpenimpulse und das Wägeergebnis bis auf eine Abweichung von 2 Takten (2 ml) erkennen.

Zur Ansteuerung der Substituatpumpe wird ein Zahlenwert für die Substituatrate in einen Analogwert entsprechend dem bei der Ansteuerung verwendeten Spannungspotential gewandelt. Da die Fördermenge einer peristaltischen Schlauchpumpe neben der Drehzahl und dem Eingangsdruck außerdem noch von der Dicke des verwendeten Schlauches abhängt, wird die von der Pumpe geförderte Menge je Zeiteinheit gemessen und als Kalibriereinheit abgelegt, wie dies vorstehend erläutert worden ist. In dieser Füllphase läuft die Pumpe mit maximaler Drehzahl, so daß nach der Kalibrierung das Betriebssystem den entsprechenden binären Einstellwert für den D/A-Wandler bei der jeweiligen Fördermenge errechnen kann. Das Schutzsystem überwacht dabei diese Drehzahl über eine auf der Pumpenmotorachse angebrachten Taktscheibe, wie dies ebenfalls vorstehend erläutert ist. Der ebenfalls vom Schutzsystem errechnete D/A-Wert wird über eine Tabelle in einen Frequenzwert gewandelt, der der Fördermenge proportional ist. Die Rückmeldung der Taktscheibe wird auf einen Zähler gegeben, der in bestimmten Zeitabständen gelesen wird. Da diese Frequenz durch die Regelung des Pumpenmotors leichten Schwingungen unterlegen ist, wird vorteilhafterweise aus mehreren Zählerzuständen, beispielsweise 8, ein Mittelwert gebildet, der mit dem abgelegten Tabellenwert verglichen wird.

Neben der Bilanzierung über die Substituat- und Ultrafiltratpumpe wird die Bilanz auf eine weitere Weise über das von der Waage abgegebene Gewicht überprüft. Da sowohl dem Betriebssystem als auch dem Schutzsystem der Gewichtszuwachs pro Zeiteinheit (Entzugsrate) bekannt ist, wird dieser vorteilhaft vom Betriebssystem und vom Schutzsystem zugleich überpüft. Bei einer Abweichung des Ist-Wertes vom berechneten Wert wird die Substituatpumpe in bestimmten Grenzen, beispielsweise ± 5 ml nachgeregelt, um eine genaue Bilanzierung über die Behandlungszeit zu erreichen. Sofern ein Abdriften von mehr als 10 ml festgestellt wird, wird die Behandlung unterbrochen.

Ein Fehler der Substitutionsrate wird folgendermaßen festgestellt:
Das Schutzsystem kennt das von der Waage abgegebene Gewicht, den Ist-Wert der Drehzahl der Substituatpumpe sowie die Substitutions- und die Ultrafiltrationsrate.

Aus der Kenntnis der Soll-Substitutionsrate kann das Schutzsystem eine Grobüberwachung der Drehzahl der Substituatpumpe vornehmen, wobei "grob" auf die Schlauchtoleranzen zurückzuführen ist, die mit ± 10 % einzukalkulieren sind.

Des weiteren ist über die Kenntnis des Waagenwerts und der Soll-Ultrafiltrationsrate eine genaue Kontrolle der Substitutionsrate möglich. Insofern würde ein Fehler in der Waagenanzeige eine Drift der Substituatpumpendrehzahl bei vorgegebener Substitutionsrate zur Folge haben.

Aufgrund der vorgegebenen engen Grenzen der Feinregulierung weigert sich das Betriebssystem, eine über diese Grenzen hinausgehende Drift durch Anpassung der Drehzahl der Substituatpumpe zu kompensieren.

Andererseits überwacht das Schutzsystem eine eventuelle Drift der Substitutionsrate durch Integration der Förderrate der Substituatpumpe.

Geht nun der durch eine zeitintegrierte Abweichung der Substitutionsrate ermittelte Wert über einen vorbestimmten Wert hinaus, beispielsweise 15 ml absolut, so wird dies als Fehler erkannt, was zur Abschaltung der Vorrichtung führt.

Insofern muß das Schutzsystem nicht die absolute Fördergenauigkeit der Substituatpumpe, sondern nur deren Drift überwachen.

Erfindungsgemäß wird neben diesen beiden vorstehend beschriebenen Prüfungen der Bilanz vorteilhafterweise eine Kontrolle der beiden Systeme, nämlich des Betriebssystems und des Schutzsystems in bestimmten Zeitabständen (Sekundenabständen) auf Funktionsfähigkeit vorgenommen. Dabei tauschen beide Systeme ihren aktuellen Statuszustand aus, wobei vorteilhafterweise eine bidirektionale Schnittstelle zu diesem Austausch eingesetzt wird. Sofern diese Querverbindung nicht aufrechterhalten werden kann bzw. erhebliche Abweichungen in den übermittelten Werten auftreten, wird die Behandlung abgebrochen.

Demzufolge wird erfindungsgemäß vorteilhafterweise das Betriebssystem als Steuereinrichtung und das Schutzsystem als Überwachungseinrichtung eingesetzt, wobei sich beide Einrichtungen wechselseitig überwachen. Demzufolge steuert das Betriebssystem die Schlauchpumpe für das Substituat, die Ultrafiltratpumpe, eine Plattenheizung, mit der die Substitutionsflüssigkeit vorgewärmt wird, die Anzeigen (Displays) und eine Eingabetastatur für die Eingabevorrichtung. Desweiteren bereitet es vorteilhafterweise das von der Waage abgegebene Wägesignal in einen verarbeitbaren Binärcode auf, der über eine gesonderte Leitung auch dem Schutzsystem als untergeordnetes Überwachungskriterium übermittelt wird.

Das Schutzsystem dagegen überwacht die Ultrafiltratpumpe, die Substituatpumpe und die vorstehend genannte Plattenheizung. Es korrespondiert außerdem im Sekundenrhythmus mit dem Betriebssystem über die CL-Schnittstelle und erkennt über Fehlsteuerungen auch einen "Ausfall" des Betriebssystems. Über diese Leitung werden auch Alarmzustände der Hämodiafiltrationsvorrichtung und eigene Fehler (Substituat, Ultrafiltrat und Heizung) übermittelt, um sie dem Bediener im Klartext anzeigen zu können.

Die Plattenheizung wird analog gesteuert und überwacht, das Betriebssystem schaltet lediglich die Heizung ein, während das Schutzsystem die Signalleitungen für Über-und Untertemperatur überprüft, wobei die Heizung bei Übertemperatur über ein Sicherheitsrelais selbst abgeschaltet wird.

Als Filter wird für die Hämodiafiltration ein Hämodialysator eingesetzt, der insbesondere eine hohe Flüssigkeitsdurchlässigkeit (high flux) aufweist und deshalb als Hämodiafilter bezeichnet wird. Für diese Zwecke ist durch die andere Kammer des Dialysators ein Dialysierflüssigkeitsweg belegt, an dessen Anfang eine Dialysierflüssigkeitsquelle vorgesehen ist. Vorteilhafterweise ist in dem Dialysierflüssigkeitsweg ein Bilanziersystem vorgesehen, den Dialysierflüssigkeitsweg und die andere Kammer gegenüber der Umgebung abschließt.

Außer für die Hämodiafiltration können das Verfahren und die Vorrichtung gemäß der Erfindung auch für die Hämofiltration und für die Plasmafiltration eingesetzt werden. Bei diesen Blutbehandlungsverfahren kommen dann als Filter Hämofilter oder Plasmafilter zum Einsatz, deren zweite Kammer lediglich mit einer Ultrafiltrationseinheit verbunden ist.

Eine Ausführungsform der Erfindung wird nachstehend anhand der Zeichnung erläutert.

Es zeigen

Fig. 1    schematisch eine Hämodiafiltrationsanordnung gemäß der Erfindung und

Fig. 2    einen Ausschnitt aus der Hämofiltrationsanordnung gemäß Fig. 1, in dem die Steuereinheit und die Überwachungseinheit gesondert gezeigt sind.

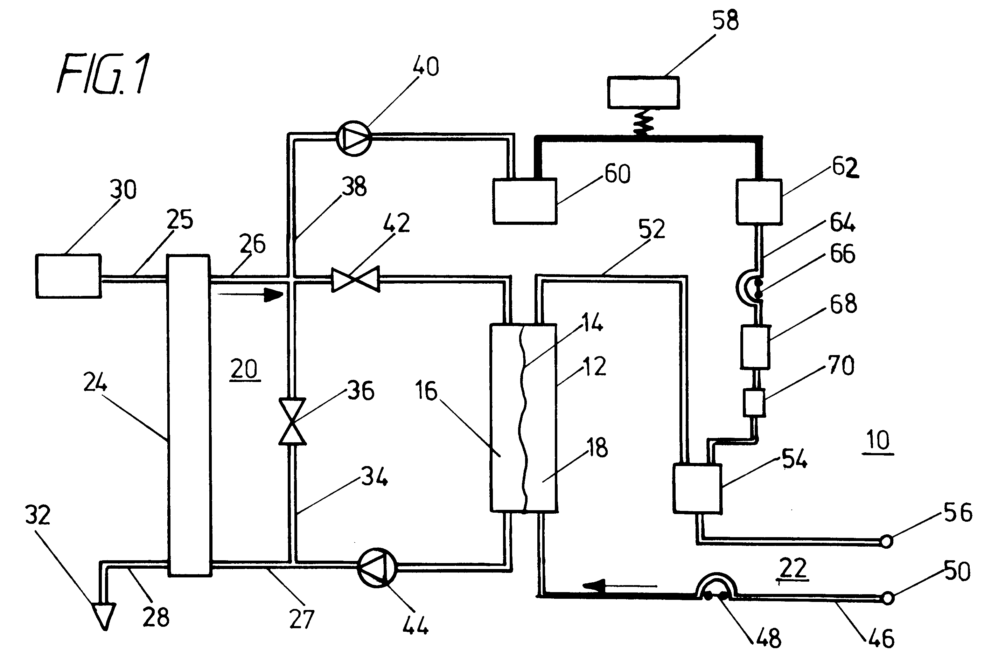

In Fig. 1 ist mit (10 ) eine Hämodiafiltrationsvorrichtung gezeigt, die aus einem Dialysator (12) besteht, der durch eine Membran (14) in eine erste Kammer (16) und eine zweite Kammer (18) geteilt ist. Durch die erste Kammer ist ein Dialysierflüssigkeitsweg (20) gelegt, während durch die zweite Kammer ein Blutweg (22) gelegt ist.

Der Dialysierflüssigkeitsweg (20) weist ein Bilanziersystem (24) auf, das einerseits mit einer Zuleitung (25,26) und andererseits einer Ableitung (27, 28) für die Dialysierflüssigkeit verbunden ist. Des weiteren wird die Dialysierflüssigkeit aus einer Dialysierflüssigkeitsquelle (30) der Zuleitung (25,26) zur Verfügung gestellt.

Ein derartiger Dialysierflüssigkeitsteil ist beispielsweise in der DE-OS 2838414 beschrieben, auf deren Offenbarung Bezug genommen wird. Zwischen dem Bilanziersystem (24), der Zuleitung (26), der ersten Kammer (16) und der Ableitung (27) liegt ein geschlossenes System vor, das zwei Bilanzkammern aufweist, die jeweils durch eine elastische Wand in zwei Kammern unterteilt sind. Beide Bilanzkammern werden wechselweise in den geschlossenen Kreislauf bzw. außerhalb des geschlossenen Kreislaufs geschaltet, wobei im letzten Fall die Bilanzkammer mit der Dialysierflüssigkeitsquelle einerseits und dem mit 32 gekennzeichneten Abfluß andererseits verbunden ist. Wie bereits festgestellt, dient der Einsatz einer derartigen Bilanziereinheit (24) zur Eröffnung eines geschlossenen Dialysierflüssigkeitskreislaufes für den Dialysator (12).

Des weiteren sind die Zuleitung (26) und die Ableitung (27) mit einer Bypassleitung (34) verbunden, in die ein Bypassventil (36) eingeschaltet ist. Des weiteren geht von der Zuleitung (26) eine Ultrafiltrationsleitung (38) ab, in die eine Ultrafiltrationspumpe (40) eingeschaltet ist.

Stromab dieser Verzweigung und der Bypassleitung (34) ist in die Zuleitung (26) ein Dialysatorventil (42) eingeschaltet, das in dem Gegentakt zum Bypassventil (36) betrieben wird.

Schließlich ist in der Ableitung (27) eine Dialysierflüssigkeitspumpe (44) vorgesehen, mit der im geschlossenen Kreislauf die Dialysierflüssigkeit umgepumpt wird.

Der Blutweg (22) besteht aus einer Blutzuleitung (46), in die eine Blutpumpe (48) eingeschaltet ist, wobei das eine Ende (50) eine nicht gezeigte Blutzugangseinrichtung aufweist, während das andere Ende mit der zweiten Kammer (18) des Dialysators (12) verbunden ist.

Der Ausgang der zweiten Kammer (18) ist mit einer Blutabzugsleitung (52) verbunden, in die eine Tropfkammer (54) eingeschaltet ist, und deren anderes Ende (56) wiederum eine nichtgezeigte Blutzugangseinrichtung aufweist.

Eine Vorrichtung dieser Art wird beispielsweise von der Anmelderin unter der Bezeichnung A2008 vertrieben und ist in der vorstehend erwähnten DE-OS 2838414 beschrieben.

Um eine Hämofiltrationsvorrichtung (10) zu bilden, ist eine Waage (58) vorgesehen, auf der ein dem geschlossenen Dialysierflüssigkeitssystem entnommenes Ultrafiltrat einerseits und eine Substitutionsflüssigkeit andererseits bilanziert erfaßt werden.

Demzufolge weist die Waage (58) einen oder mehrere Behälter (60) zur Aufnahme von Ultrafiltrat und einen oder mehrere Behälter (62) zur Abgabe von Substitutionsflüssigkeit auf.

Dabei ist das Ende der UF-Leitung (38) mit dem UF-Behälter (60) verbunden, während vom Substitutionsflüssigkeitsbehälter (62) eine Substituatleitung (64) abgeht, in die eine Substituatpumpe (66), eine Heizeinrichtung (68) und ein Temperatursensor (70) eingeschaltet sind. Das andere Ende der Substituatleitung (64) ist mit der Tropfkammer (54) verbunden.

Bei der letztgenannten Verbindung handelt es sich um eine sog. Postdilution, d.h. das Blut wird stromab des Dialysators (12) mit der Substitutionsflüssigkeit verdünnt.

Andererseits ist jedoch aber auch die Verbindung der Substituatleitung (64) mit der Blutzuleitung (46) (Predilution) möglich.

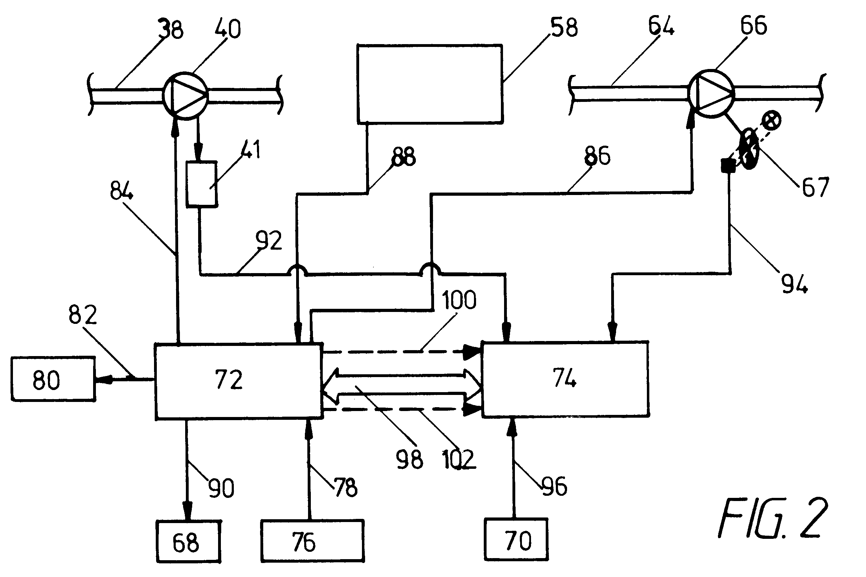

In Fig. 2 ist dargestellt, wie die Ultrafiltrationspumpe (40), die Waage (58), die Substituatpumpe (66) und die Heizeinrichtung (68) mit einer Steuereinheit (72) und einer Überwachungseinheit (74) verbunden sind.

Die Steuereinheit (72) ist zunächst mit einer Eingabevorrichtung (76) über die Leitung (78) verbunden. Über diese Eingabevorrichtung (76) können die Behandlungszeit T, das zu entnehmende Ultrafiltrat $Q_{UF}$ und die abgegebene Substituatmenge $Q_{Sub}$, sofern notwendig, eingegeben werden.

Die Steuereinheit (72) ist weiterhin mit einer Anzeigeeinheit (80) über die Leitung (82) verbunden, mit der die eingegebenen Werte bzw. die aktuellen Daten angezeigt bzw. abgefragt werden können.

Des weiteren ist die Steuereinheit (72) über die Steuerleitung (84) mit der Ultrafiltrationspumpe (40) und über die Steuerleitung (86) mit der Substituatpumpe (66) verbunden. Schließlich wird über die Signalleitung (88) das von der Waage (58) abgegebene Signal entgegengenommen.

Des weiteren ist die Steuereinheit (72) mit der Heizeinrichtung (68) über die Leitung (90) verbunden.

Die Überwachungseinheit (74) ist über eine Signalleitung (92) mit der Ultrafiltrationspumpe (40), über eine Signalleitung (94) mit der Substituatpumpe (66) und über eine Signalleitung (96) mit dem Temperatursensor (70) verbunden. Des weiteren sind die Steuereinheit (72) und die Überwachungseinheit (74) über eine bidirektionale Leitung (98) direkt verbunden und tauschen über diese Leitung ihre Daten aus.

Schließlich werden über die gestrichelt dargestellten Übermittlungsleitungen (100 und 102) die von der Waage (58) bzw. Eingabevorrichtung (76) in die Steuereinheit (72) eingegebenen Daten an die Überwachungseinheit (74) weitergegeben.

Die Steuereinheit (72) und die Überwachungseinheit (74) können in Form einer üblichen elektronischen Schaltung aufgebaut sein. Vorzugsweise sind sie jedoch in Form programmierbarer Mikroprozessoren aufgebaut.

Die Ultrafiltrationspumpe (40) ist im Beispielsfalle eine taktgesteuerte Hubkolben- oder Membranpumpe, die je Hub eine bestimmte Menge Flüssigkeit fördert. Die Frequenz, mit der die Ultrafiltrationspumpe (40), die im Beispielsfall auch aus zwei oder mehreren Pumpeneinheiten aufgebaut sein kann, betrieben wird, wird von der Steuereinheit (72) entsprechend der Ultrafiltrationsrate aus $Q_{UF}$ und T errechnet, bestimmt. Die Überwachungseinheit (74) übernimmt das Rückmeldesignal der Ultrafiltrationspumpe (40) aus einer Rückmeldeeinrichtung (41) und vergleicht dies mit dem aus den Eingabewerten errechneten Wert sowie mit dem über die Leitungen 88 und 100 abgegebenen Wägewert der Waage (58).

Dabei errechnen vorteilhafterweise die beiden Steuer- und Überwachungseinrichtungen (72 und 74) unabhängig voneinander in Abhängigkeit von der Behandlungsdauer T, $Q_{Sub}$ und $Q_{UF}$ die Ultrafiltrationsrate sowie die Substituatrate, mit der die beiden Pumpen (40 und 66) betrieben werden müssen. Über die bidirektionale Leitung 98 tauschen die beiden Einrichtungen 72 und 74 jeweils in bestimmten Zeitabständen diese Daten aus und vergleichen diese miteinander auf Plausibilität.

Des weiteren ist die Substituatpumpe (66) als peristaltische Pumpe ausgestaltet, die wegen ihrer relativ großen Gangungenauigkeit und des in sie eingelegten Schlauchsystems, das gewissen Fertigungstoleranzen unterlegen ist, jeweils zu Beginn der Behandlung geeicht werden muß, wie dies bereits vorstehend beschrieben worden ist, worauf Bezug genommen wird. In Fig. 2 ist ein optoelektronisches Überwachungssystem (67) dafür gezeigt, das aus einer auf der Pumpenantriebsachse des Substituatpumpe (66) angeordneten Strichscheibe einer Lichtquelle und einem Lichtdetektor besteht.

Schließlich wird das Wägesignal in der Steuereinheit (72) - wie bereits vorstehend erwähnt - digital aufbereitet und danach über die Leitung (100) an die Überwachungseinrichtung (74) abgegeben.

Nach der Eichung der Substituatpumpe (66) kann die Überwachungseinheit (74) über das durch die Leitung (94) abgegebene Signal die von der Substituatpumpe (66) abgegebene Substituatmenge berechnen und löst bei einer vorgegebenen Abweichung von ± 5 ml eine Nachregelung der Substituatpumpe (66) aus, um eine genaue Bilanzierung über die Behandlungszeit zu erreichen. Diese Nachregulierung hat jedoch ihre Grenze bei einem Abdriften von mehr als 10 ml, so daß die Behandlung hierdurch unterbrochen wird.

Wie bereits vorstehend erwähnt, errechnet die Steuereinheit (72) aus der vorgegebenen Ultrafiltratrate (ml/min) eine Frequenz, mit der die Ultrafiltrationspumpe (40) angesteuert wird. Diese Ansteuerfrequenz wird mit Hilfe eines Timers gebildet, dessen Preset-Werte in einer Tabelle in 1 ml-Stufen abgelegt sind. Diese vom Timer erzeugte, der Ultrafiltrationsrate proportionale Frequenz steht am Ausgang der Steuereinheit (72) zur Verfügung.

Die Überwachungseinheit (74) übernimmt das Rückmeldesignal von der Rückmeldeinrichtung (41) wobei die Rückmeldeimpulse auf ein Zähler gegeben werden, und errechnet in Minutenabständen den aktuellen Status.

Die Substituatpumpe (66) wird dagegen kontinuierlich betrieben, üblicherweise mit einem Spannungspotential von 0-10 V. Dementsprechend wird der in der Steuereinrichtung (72) vorgegebene Zahlenwert für die Substituatrate in einen Analogwert umgewandelt, wobei ein 8 Bit D/A-Wandler eingesetzt werden kann, was einer Ansteuerung in 39 mV-Schritten entspricht. Nach der Eichung der Substituatpumpe (66) mit maximaler Drehzahl kann die Steuereinheit (72) den jeweiligen binären Einstellwert für den D/A-Wandler bei der jeweiligen Fördermenge der Substituatpumpe (66) errechnen.

Die Überwachungseinheit (74) überwacht dagegen diese Drehzahl durch die auf der Pumpenmotorachse angebrachte Taktscheibe und das optoelektronische Meßsystem, wobei der errechnete D/A-Wert über eine Tabelle in einen Frequenzwert gewandelt wird, der der Fördermenge proportional ist. Die Rückmeldung dieser Taktscheibe wird auf einen Zähler gegeben, der in Sekundenabständen gelesen wird, wobei vorteilhafterweise aus mehreren Zählerzuständen ein Mittelwert gebildet wird, der mit dem Tabellenwert verglichen wird.

Neben der Bilanzierung über die Förderraten der Ultrafiltrationspumpe (40) und der Substituatpumpe (66) wird von der Steuereinheit (72) und der Überwachungseinheit (74) die Bilanz auf einem zweiten Weg über das Gewicht überprüft. Hierzu wird das aktuelle Wägesignal, das der bis zum Meßzeitpunkt tatsächlich entnommenen Ultrafiltratmenge entspricht, mit dem aus den eingegebenen Daten $Q_{SUb}$, $Q_{UF}$ und der Behandlungsdauer T errechneten Gewichtswert verglichen. Bei Abweichungen in Grenzen von ± 5 ml wird die Substituatpumpe (60) nachgeregelt. Bei einem Abdriften von mehr als 10 ml wird die Behandlung unterbrochen.

Die Heizeinrichtung (68) wird analog von der Steuereinheit (72) gesteuert und von der Überwachungseinheit (74) mittels des Temperatursensors (70) innerhalb vorstehender Temperaturgrenzen zwischen 36 und 42° C überwacht.

**Patentansprüche**

1.   Blutbehandlungsvorrichtung mit einem Filter, der durch eine Membran (14) in zwei Kammern (16, 18) geteilt ist und durch dessen eine Kammer (18) ein extrakorporaler Blutkreislauf (22) gelegt und dessen andere Kammer (16) mit einer Ultrafiltrationseinheit verbunden ist, mit der mittels einer Ultrafiltratpumpe (40) aus der anderen Kammer (16) Ultrafiltrat entnommen wird, mit einer Substitutionseinheit, mit der mittels einer Substituatpumpe (66) dem Blutkreislauf (22) eine Substitutionsflüssigkeit zugeführt wird, mit einer Waage (58), die die zugeführte Ultrafiltratmenge und die abgeförderte Substitutionsflüssigkeitsmenge gegeneinander bilanziert, indem der Ultrafiltratbehälter (60) und der Substitutionsflüssigkeitsbehälter (62) mit ihrem jeweiligen Inhalt gemeinsam gewogen werden, sowie mit einer Steuereinheit (72) zur Steuerung der Ultrafiltratpumpe (40) und der Substituatpumpe (66), dadurch gekennzeichnet, daß

     a. die Ultrafiltratpumpe (40) taktbetrieben ist und je Takt eine bestimmte Menge Flüssigkeit fördert,
     b. die Waage (58) mit einer Überwachungseinheit (74) verbunden ist,
     c. die Ultrafiltratpumpe (40) über eine Rückmeldeeinrichtung (41) mit der Überwachungseinheit (74) verbunden ist und die Rückmeldeeinrichtung (41) die Taktimpulse der Ultrafiltratpumpe (40) an die Überwachungseinrichtung (74) meldet, welche aus der Summe der Taktimpulse und dem Pumpvolumen der Ultrafiltratpumpe (40) die von der Ultrafiltratpumpe (40) geförderte Ultrafiltratmenge errechnet und vor Beginn der Blutbehandlung den errechneten Wert mit dem von der Waage ermittelten Wert vergleicht und
     d. eine Eingabevorrichtung (76) mit der Überwachungseinheit (74) verbunden ist, über die die zu ultrafiltrierende Menge $Q_{UF}$, die zu substituierende Menge $Q_{Sub}$ und die Behandlungszeit T eingebbar sind, und daß die Überwachungseinheit (74) aus den Parametern $Q_{UF}$, $Q_{Sub}$ und T in bestimmten Zeitabständen die momentan auf der Waage (58) befindliche Menge errechnet und den errechneten Wert mit dem von der Waage (58) gelieferten Wägeergebnis vergleicht und bei einem Abweichen der beiden Werte um ein vorbestimmtes Maß einen Alarm auslöst und die Behandlung unterbricht.

2.   Vorrichtung nach Anspruch 1, dadurch gekennzeichnet, daß die Substituatpumpe (66) eine peristaltische Pumpe ist, der eine optoelektronische Überwachungseinrichtung (67) zugeordnet ist.

3.   Vorrichtung nach Anspruch 2, dadurch gekennzeichnet, daß die optoelektronische Überwachungseinrichtung (67) eine Strichscheibe aufweist, die auf der Pumpenantriebsachse angeordnet ist und der eine Lichtquelle und ein Lichtdetektor zugeordnet sind, und daß die entwickelten Signale von der Überwachungseinheit (74) gezählt werden.

4. Vorrichtung nach Anspruch 1, dadurch gekennzeichnet, daß die Ultrafiltratpumpe (40) eine Förderungsgenauigkeit von weniger als 1% und die Waage eine Wägeungenauigkeit von weniger als 1% aufweisen.

5. Vorrichtung nach Anspruch 1, dadurch gekennzeichnet, daß die Steuereinheit (72) und die Überwachungseinheit (74) über eine bidirektionale Leitung (98) miteinander verbunden sind und über diese Leitung im Kreuzvergleich die aus den Parametern $Q_{Sub}$, $Q_{UF}$ und T errechneten Daten austauschen.

6. Vorrichtung nach Anspruch 1, dadurch gekennzeichnet, daß der Filter ein Hämodialysator oder ein Hämodiafilter (12) ist, durch dessen andere Kammer (16) ein Dialysierflüssigkeitsweg (20) gelegt ist, der mit einer Dialysierflüssigkeitsquelle (30) verbunden ist und in dem ein Bilanziersystem (24) vorgesehen ist, das einen geschlossenen Dialysierflüssigkeitskreislauf bildet.

7. Vorrichtung nach Anspruch 1, dadurch gekennzeichnet, daß der Filter ein Hämofilter oder ein Plasmafilter ist.

**Claims**

1. Blood treatment apparatus comprising a filter which is divided by a membrane (14) into two chambers (16, 18) said first chamber (18) is connected to an extracorporeal blood circuit (22) and said other chamber (16) is connected to an ultrafiltration unit with which by means of an ultrafiltrate pump (40) ultrafiltrate is withdrawn from the other chamber (16), a substitution unit with which by means of a substitute pump (66) a substitute solution is supplied to the blood circuit (22), a balance means (58) which balances the supplied ultrafiltrate amount and the removed ultrafiltrate amount against each other by weighing jointly the ultrafiltrate container (60) and the substitute solution container (62) with their respective content, and a control unit (72) for controlling the ultrafiltrate pump (40) and the substitute pump (66), characterized in that
   a) the ultrafiltrate pump (40) is operated in cycles and delivers a predetermined amount of solution per cycle,
   b) the balance means (58) is connected to a monitoring unit (74),
   c) the ultrafiltrate pump (40) is connected via an acknowledgement means (41) to the monitoring unit (74) and the acknowledgement means (41) reports the clock pulses of the ultrafiltrate pump (40) to the monitoring means (74) calculating from the sum of the clock pulses and the pump volume of the ultrafiltrate pump (40) the ultrafiltrate amount delivered by the ultrafiltrate pump (40) and comparing before the start of the blood treatment the calculated value with the value determined by the balance means and
   d) an input device (76) is connected to the monitoring unit (74) via which the amount $Q_{UF}$ to be ultrafiltrated, the amount $Q_{sub}$ to be substituted and the treatment time T can be entered, and that the monitoring unit (74) calculates from the parameters $Q_{UF}$, $Q_{sub}$ and T at predetermined intervals of time the amount momentarily disposed on the balance means (58) and compares the calculated value with the weight result furnished by the balance means (58) and in the event of a deviation of the two values by a predetermined extent activates an alarm and interrupts the treatment.

2. Apparatus according to claim 1, characterized in that the substitutate pump (66) is a peristaltic pump with which an optoelectronic monitoring device (67) is associated.

3. Apparatus according to claim 2, characterized in that the optoelectronic monitoring device (67) is a slit disc which is arranged on the pump drive shaft and with which a light source and a light detector are associatd and that the signals generated are counted by the monitoring unit (74).

4. Apparatus according to claim 1, characterized in that the ultrafiltrate pump (40) has a delivery inaccuracy of less than 1% and the balance means a weighing inaccuracy of less than 1%.

5. Apparatus according to claim 1, characterized in that the control unit (72) and the monitoring unit (74) are connected together via a bidirectional line (98) and exchange via said line in cross comparison the data calculated from the parameters $Q_{UF}$, $Q_{sub}$ and T.

6. Apparatus according to claim 1, characterized in that the filter is a hemodialyzer or a hemodiafilter (12) through the other chamber (16) of which a dialysis solution path (20) is disposed which is connected to a dialysis solution source (30) and in which a balancing system (24) is provided which forms a closed dialysis solution circuit.

7. Apparatus according to claim 1, characterized in that the filter is a hemofilter or a plasma filter.

**Revendications**

1. Dispositif pour le traitement sanguin, comprenant un filtre, divisé en deux chambres (16,18) au moyen d'une membrane (14), et dans une première chambre (18) duquel est placé un circuit sanguin extracorporel (22) et dont la deuxième chambre (16) est reliée à une unité d'ultrafiltration, à l'aide de laquelle, au moyen d'une pompe à ultrafiltrat (40) un ultrafiltrat est prélevé de la deuxième chambre (16), avec une unité de substitution, à l'aide de laquelle, au moyen d'une pompe à substituat (66), un liquide de substitution est amené au circuit sanguin (22), avec une balance (58), qui équilibre la quantité d'ultrafiltrat amenée et la quantité de liquide de substitution évacuée, le contenu respectif du récipient à ultrafiltrat (60) et du récipient à liquide de substitution (62) étant pesés conjointement, ainsi qu'avec une unité de commande (72), pour assurer la commande de la pompe à ultrafiltrat (40) et de la pompe à substituat (66), caractérisé en ce que
   a. la pompe à ultrafiltrat (40) est commandée séquentiellement et véhicule à chaque séquence une quantité déterminée de liquide,
   b. la balance (58) est reliée à une unité de surveillance (74),
   c. la pompe à ultrafiltrat (40) est reliée par un dispositif de retour d'information (41) à l'unité de surveillance (74) et le dispositif de retour d'information (41) communiquant les informations des impulsions séquencées de la pompe à ultrafiltrat (40) au dispositif de surveillance (74), qui, à partir de la somme des impulsions cadencées et du volume de pompage de la pompe à ultrafiltrat (40), calcule la quantité d'ultrafiltrat véhiculée par cette dernière et compare, avant le début du traitement sanguin, la valeur calculée à la valeur déterminée par la balance, et
   d. un dispositif d'introduction (76), auquel est relié l'unité de surveillance (74), par l'intermédiaire duquel la quantité $Q_{UF}$ à soumettre à une ultrafiltration, la quantité $Q_{SUB}$ à substituer et le temps de traitement T sont susceptibles d'être introduits, et en ce que l'unité de surveillance (74), à partir des paramètres $Q_{UF}$, $Q_{SUB}$ et T, à des intervalles de temps déterminés, calcule la quantité qui se trouve momentanément sur la balance (58) et compare la valeur calculée au résultat de pesée fourni par la balance (58) et, en cas d'écart des deux valeurs de la valeur d'une quantité prédéterminée déclenche une alarme et interrompt le traitement.

2. Dispositif selon la revendication 1, caractérisé en ce que la pompe à substituat (66) est une pompe péristaltique, à laquelle est associée un dispositif de surveillance optoélectronique (67).

3. Dispositif selon la revendication 2, caractérisé en ce que le dispositif de surveillance optoélectronique (67) présente un disque gradué, disposé sur l'axe d'entraînement de la pompe et auquel sont associés une source de lumière et un détecteur de lumière, et en ce que les signaux produits sont comptés par l'unité de surveillance (74).

4. Dispositif selon la revendication 1, caractérisé en ce que le débit refoulé par la pompe à ultrafiltrat (40) est d'une précision de moins de 1% et la balance présente une précision de pesage de moins de 1%.

5. Dispositif selon la revendication 1, caractérisé en ce que l'unité de commande (72) et l'unité de surveillance (74) sont reliées par l'intermédiaire d'une ligne bidirectionnelle (98) et échangent par l'intermédiaire de cette ligne, suivant une comparaison croisée, les données calculées à partir des paramètres $Q_{SUB}$, $Q_{UF}$ et T.

6. Dispositif selon la revendication 1, caractérisé en ce que le filtre est un hémodialyseur ou un hémodiafiltre (12), dans la deuxième chambre duquel est placée une voie de liquide de dialyse (20), qui est reliée à une source de liquide de dialyse (30) et dans laquelle est prévue un système d'équilibrage (24), qui forme un circuit fermé de liquide de dialyse.

7. Dispositif selon la revendication 1, caractérisé en ce que le filtre est un hémofiltre ou un filtre à plasma.

FIG.1

EP 0 321 754 B1

FIG. 2

EP 0 321 754 B1